# EUROPÄISCHE PATENTSCHRIFT

(11) **EP 1 224 157 B1**
(45) Veröffentlichungstag und Bekanntmachung des Hinweises auf die Patenterteilung: **22.12.2004**
(21) Anmeldenummer: 00966108.3
(22) Anmeldetag: 04.10.2000
(51) Int. Cl.: C07C 41/09, C07C 43/20

(54) **VERFAHREN ZUR HERSTELLUNG VON ARYLALKYLETHERN**
METHOD FOR PRODUCTION OF ARYL ALKYL ETHERS
PROCEDE POUR PRODUIRE DES ETHERS D'ARYLALKYLE

(30) Priorität: 13.10.1999 DE 19949319
(43) Veröffentlichungstag der Anmeldung: 24.07.2002
(73) Patentinhaber: Symrise GmbH & Co. KG, 37603 Holzminden (DE); Rütgers VFT AG, 44579 Castrop-Rauxel (DE)
(72) Erfinder: BILLER, Efim, A-1190 Wien (AT); FUHRMANN, Edgar, 44579 Castrop-Rauxel (DE); HAGENA, Detlef, 37671 Höxter (DE); JANNECK, Heinz, Günter, 45711 Datteln (DE); TALBIERSKY, Jörg, 46282 Dorsten (DE); WALTHER, Lutz, 37603 Holzminden (DE)
(74) Vertreter: Eisenführ, Speiser & Partner
(86) Internationale Anmeldenummer: PCT/EP2000/009699
(87) Internationale Veröffentlichungsnummer: WO 2001/027060

(56) Entgegenhaltungen:
- EP-A- 0 046 719
- EP-A- 0 076 221
- US-A- 3 911 022
- CHEMICAL ABSTRACTS, vol. 75, no. 7, 16. August 1971 (1971-08-16) Columbus, Ohio, US; abstract no. 48669a, S. SENOO: "Ether compounds" Seite 329; Spalte 1; XP002157311 & JP 71 011494 A (ASAHI CHEMICAL INDUSTRY) 24. März 1971 (1971-03-24)

## Beschreibung

Die Erfindung betrifft ein Verfahren zur Herstellung von Arylalkylethern, in dem Hydroxyaromaten mit einem Alkohol in Gegenwart eines Katalysators umgesetzt werden.

Arylalkylether wie das Anisol (Methylphenylether) werden beispielsweise unter anderem als Zwischenprodukte bei Arzneistoff- und Riech-/Geschmackstoffsynthesen benötigt.

Die EP 076 221 A1 beschreibt die Veretherung von Hydroxyaromaten mit Alkoholen in Gegenwart eines Carbonsäuresalzes und eines Metallhalogenids.

Ein weiteres Verfahren dieser Art ist in der JP 43-45852 beschrieben, welches in Form eines Batch-Verfahrens durchgerührt wird. Dabei steigt im Verlauf der zehnstündigen Umsetzung bei 240°C in einem Autoklaven die Produktkonzentration auf 20,6 % nach 1 Stunde, bis 93% nach 10 Stunden an. Die Umsetzung wird bei 180 bis 280°C, vorzugsweise bei etwa 240°C, durchgeführt, wobei Alkohole vorzugsweise mindestens in gleicher Stoffmenge eingesetzt werden wie die Phenole. Der Katalysator wird in den Beispielen in einer Menge von etwa 1,75 Gewichtsteilen auf 1 Gewichtsteil Phenol eingesetzt. Nach zweistündiger Reaktionsdauer beträgt die Anisolausbeute etwa 42,6 % und kann nach zehnstündiger Reaktion und einem großen Überschuss Methanol auf etwa 87 % gesteigert werden.

Nachteilig an diesem Verfahren, insbesondere bei dem zur Steigerung der Ausbeute erforderlichen hohen Überschuss an Alkohol, ist die störende Bildung von kernalkylierten Produkten sowie von Dialkylethern. Weiterhin ist die eingesetzte Katalysatorkonzentration so hoch, dass weder im Ausgangsgemisch, noch im Katalysat bei Normaldruck eine homogene Lösung erreichbar ist. Dieses Zweiphasengemisch mit Salzanteil ist für eine kontinuierliche Reaktionsführung nicht einsetzbar. Ferner ist vor der Destillation ein weiterer Verfahrensschritt zur Salzabtrennung erforderlich, der eine Filtration und Extraktion umfasst.

Der Erfindung lag deshalb die Aufgabe zugrunde, ein Verfahren zur Herstellung von Arylalkylethern bereitzustellen, in dem wesentlich höhere Ausbeuten erzielt werden und die Bildung von kemalkylierten Produkten und Dialkylethem deutlich verringert ist.

Gelöst wird diese Aufgabe durch ein Verfahren zur Herstellung von Arylalkylethern, in dem ein Hydroxyaromat mit einem Alkohol in Gegenwart eines Katalysators umgesetzt wird, wobei man ein bei Normaltemperatur homogenes, Hydroxyaromat, Alkohol und Katalysator enthaltendes Gemisch in den Reaktor einleitet, bei einer Temperatur von 250 bis 370°C umsetzt und die Produktkonzentration im Reaktionsgemisch derart einstellt, dass der Katalysator nicht ausfällt, wobei das Verfahren kontinuierlich durchgeführt wird und das Molverhältnis Katalysator zu Hydroxyaromat 1:32 - 1:60 beträgt.

Gegenüber dem gattungsgemäßen kontinuierlichen Verfahren der JP 43-45852 wird erfindungsgemäß eine Steigerung der Raum/Zeit-Ausbeute um Faktor >9 erzielt. Obwohl die Umsetzung bei höheren Temperaturen üblicherweise zu einem vermehrten Anfall von kemalkylierten Verbindungen führt, ist dies im erfindungsgemäßen Verfahren nicht der Fall. Überraschenderweise ist die Selektivität des erfindungsgemäßen Verfahrens derart erhöht, dass die Bildung von Dialkylethern praktisch unterbleibt. Der Katalysator im Reaktionsgemisch wirkt sich darüber hinaus nicht störend auf die anschließende Destillation aus.

Erfindungsgemäß einzusetzende Hydroxyaromaten sind solche der allgemeinen Formel HO-Ar-(R)ₙ, worin Ar einen substituierten oder nicht substituierten Benzolring oder substituiertes oder nicht substituiertes aromatisches Ringsystem wie Naphthalin, Anthracen oder Phenantren bedeutet; die Substituenten R oder der Substituent R gleich oder verschieden sind und eine Hydroxylgruppe, einen geradkettigen oder verzweigten Alkylrest oder Alkenylrest mit 1 bis 6 Kohlenstoffatomen, einen gegebenenfalls substituierten Phenylrest, einen gegebenenfalls substituierten Cycloalkylrest, einen Phenylalkylrest, dessen Alkylrest 1 bis 4 Kohlenstoffatome umfasst, bedeutet(en) und n eine ganze Zahl von 1 bis 5 ist. Besonders bevorzugt werden beispielsweise Phenol, p-Kresol, Xylenol, 2-Naphthol, Brenzkatechin, Resorcin und Hydrochinon. Hervorzuheben ist auch, dass die Alkylgruppen an Hydroxyaromaten unter den geschilderten Reaktionsbedingungen sich völlig immobil verhalten.

In dem erfindungsgemäßen Verfahren einzusetzende Alkohole sind primäre, sekundäre und tertiäre Alkohole, wie Methanol, Ethanol, n-Propanol, Isopropanol, die Butanole 1-Butanol, 2-Butanol, tert.-Butanol, n-Pentanol und dessen verzweigte Isomere und Benzylalkohol.

Das Molverhältnis Hydroxyaromat zu Alkohol kann vorzugsweise auf 0,5:1, insbesondere 1:1, eingestellt werden.

Erfindungsgemäß geeignete Katalysatoren sind aromatische Carbonsäuren wie Benzoesäure und Phthalsäuren sowie deren alkylierte Derivate und aliphatische Carbonsäuren, wie Essigsäure, Propionsäure und substituierte Fettsäuren und deren Salze. Substituierte Fettsäuren sind beispielsweise solche mit 8 bis 20 Kohlenstoffatomen. Ferner geeignet sind Arylalkylcarbonsäuren, wie Phenylbenzoesäure. Gegenionen sind beispielsweise Alkalimetalle und Erdalkalimetalle, vorzugsweise Natrium, Kalium, Lithium, Calcium, Magnesium und Barium.

Die Katalysatorkonzentration kann vorzugsweise derart eingestellt werden, dass das Molverhältnis Katalysator zu Hydroxyaromat 1:32 bis 1:60, beispielsweise auch 1:40 beträgt.

Das erfindungsgemäße Verfahren kann als Batchverfahren oder kontinuierlich durchgeführt werden, wobei die kontinuierliche Verfahrensführung bevorzugt wird. Es wurde beobachtet, dass bei der kontinuierlichen Verfahrensweise eine sehr viel bessere Volumenausnutzung erfolgt, weil gegenüber dem Batchverfahren insbesondere bei der Verwendung von Methanol dessen Konzentration in der Flüssigphase sehr viel höher ist als in der Gasphase.

Vorzugsweise wird das Verfahren in einem Rohrreaktor durchgeführt. Das in den Reaktor eingespeiste Gemisch ist bei Normaldruck eine homogene Lösung. Die Temperatur im Reaktor, bei der die Umsetzung durchgeführt wird, kann 250 bis 370°C, vorzugsweise mindestens 275°C betragen.

Die Verweildauer der Ausgangsverbindungen oder des Produkts im Reaktor wird durch die eingespeiste Menge der Ausgangsmaterialien geregelt. Die Einspeisemenge wird vorzugsweise derart eingestellt, dass der Katalysator nicht ausfällt und dass ein Umsatz von maximal etwa 40 % Reaktionsprodukt, bezogen auf das Gewicht des eingesetzten Alkylaromats, erhalten wird. Vorzugsweise beträgt der Umsatz 35 bis 40 % Produkt, bezogen auf den Hydroxyaromat. Diese Verfahrensweise ist insbesondere deshalb vorteilhaft, weil das Produkt destillierbar bleibt. Dies ist gegenüber den bekannten Verfahren vorteilhaft, weil an dieser Stelle der Verfahrensführung keine Abtrennung des Katalysators erforderlich ist, sondern gleich destilliert werden kann. Ferner ist die Raum-/Zeit-Ausbeute bei der kontinuierlichen Fahrweise gegenüber höheren Umsätzen deutlich verbessert.

Die Herstellung von Anisol ist nachfolgend beispielhaft für das erfindungsgemäße Verfahren beschrieben. Die hier beschriebene Verfahrensführung gilt aber im Prinzip auch für die Herstellung anderer nach dem erfindungsgemäßen Verfahren erhältlicher Produkte.

Die Ausgangssubstanzen Phenol, Methanol und Kaliumbenzoat werden gemischt, wobei eine homogene Lösung entsteht, die anschließend kontinuierlich durch einen Rohrreaktor mit einem Volumen von 1520 ml gepumpt wird. Das Ausgangsgemisch enthält beispielsweise 71,27 Massen-% Phenol, 24,26 Massen-% Methanol, 2,92 Massen-% Benzoesäure und 1,56 Massen-% Kaliumhydroxid. Die Temperatur im Reaktor beträgt 340 °C, der Druck etwa 80 bar. Während der Reaktion entstehen in sehr geringen Mengen Dimethylether und Kohlendioxid als gasförmige Produkte, die in einer Abgasfalle gesammelt und anschließend verbrannt werden. Die Produktkonzentration im Reaktor wird auf 40 Gew.-%, bezogen auf die eingespeiste Menge des Phenols, eingestellt. Das dem Reaktor entnommene, das Produkt enthaltene Gemisch ist so polar, dass der Katalysator nicht ausfällt. Es wird auf etwa 50°C abgekühlt, entspannt und in einem Zwischenbehälter gesammelt.

Das Katalysat wird mit Hilfe einer Destillation aufgearbeitet, Zunächst wird das nicht umgesetzte Methanol zurückgewonnen. Danach wird als Kopfprodukt ein Azeotrop aus Anisol und Wasser abgezogen. Nach der Phasentrennung zwischen Anisol und Wasser wird das Anisol kontinuierlich zum Destillationskopf zurückgeführt. Dieser Vorgang wird solange durchgeführt bis das gesamte Wasser azeotrop ausgekreist wurde. Danach erhält man als Kopfprodukt ein wasserfreies Anisol in einer Reinheit von 99,9 %. Nach weitestgehender Abtrennung des Anisols wird die Destillation beendet. Der homogene Rückstand enthält den gesamten Katalysator, nicht umgesetztes Phenol sowie Spuren Anisol. Diese Lösung wird als Rohstoff für die Anisolherstellung nach Ergänzung mit Methanol und Phenol vollständig recycelt.

### Beispiel 1

### Herstellung von 2-Methoxynaphthalin (Nerolin)

Die Ausgangssubstanzen 2-Naphthol, Methanol und Kaliumbenzoat werden gemischt, wobei eine homogene Lösung entsteht, die anschließend kontinuierlich durch einen Rohrreaktor mit einem Volumen von 1520 ml gepumpt wird. Das Ausgangsgemisch enthält 49,4 Massen-% 2-Naphthol, 49,3 Massen-% Methanol, 0,92 Massen-% Benzoesäure und 0,49 Massen-% Kaliumhydroxid. Die Temperatur im Reaktor beträgt 320°C, der Druck etwa 100 bar. Die Einspeisemenge wird so eingestellt, dass die Verweilzeit im Reaktor etwa 5 Stunden beträgt. Das Reaktorprodukt wird in einem Nachkühler auf etwa 60°C abgekühlt und danach auf Umgebungsdruck entspannt.

Bei den oben genannten Versuchsbedingungen erhält man ein Reaktionsprodukt, welches etwa 35,5 Massen-% 2-Methoxynaphthalin enthält. Weiterhin enthält es nicht umgesetztes Methanol, 2-Naphthol, Kaliumbenzoat und Reaktionswasser aus der Veretherung. Das Katalysat wird vergleichbar mit der Anisolherstellung durch Destillation aufgearbeitet. Zunächst wird das nicht umgesetzte Methanol abgenommen. Danach wird Wasser abgezogen und danach ein wasserhaltiger Vorlauf von 2-Methoxy-naphthalin, welcher zur Redestillation wiederverwendet werden kann. Als nächste Fraktion enthält man ein 2-Methoxynaphthalin mit einer Qualität von mindestens 99 Massen-%. Nach weitestgehender Abtrennung des 2-Methoxynaphthalins wird die Destillation beendet.

Der homogene Rückstand enthält den gesamten Katalysator, nicht umgesetztes 2-Naphthol sowie Spuren von 2-Methoxynaphthalin. Diese Lösung wird als Rohstoff zur 2-Methoxynaphthalinherstellung nach Ergänzung mit Methanol und 2-Naphthol vollständig recycelt.

### Beispiel 2

### Herstellung von 4-Methylanisol

Die Ausgangssubstanzen p-Cresol, Methanol und Kaliumbenzoat werden gemischt, wobei eine homogene Lösung entsteht, die anschließend kontinuierlich durch einen Rohrreaktor mit einem Volumen von 1520 ml gepumpt wird. Das Ausgangsgemisch enthält 73,7 Massen-% p-Cresol, 21,9 Massen-% Methanol, 2,9 Massen-% Benzoesäure und 1,55 Massen-% Kaliumhydroxid. Die Temperatur im Reaktor beträgt 340°C, der Druck etwa 80 bar. Die Einspeisemenge wird so eingestellt, dass die Verweilzeit im Reaktor ca. 5 Stunden beträgt. Das Reaktorprodukt wird in einem Nachkühler auf etwa 50°C abgekühlt und danach auf Umgebungsdruck entspannt.

Bei den oben genannten Versuchsbedingungen erhält man ein Reaktionsprodukt, welches etwa 36,3 Massen-% 4-Methylanisol enthält. Weiterhin enthält es nicht umgesetztes Methanol, p-Cresol, Kaliumbenzoat und Reaktionswasser aus der Veretherung. Das Katalysat wird vergleichbar mit der Anisolherstellung durch Destillation aufgearbeitet. Zunächst wird das nicht umgesetzte Methanol abgenommen. Danach wird Wasser abgezogen und danach ein wasserhaltiger Vorlauf von 4-Methylanisol, welcher zur Redestillation wiederverwendet werden kann. Als nächste Fraktion enthält man ein 4-Methylanisol mit einer Qualität von mindestens 99 Massen-%. Nach weitestgehender Abtrennung des 4-Methylanisols wird die Destillation beendet.

Der homogene Rückstand enthält den gesamten Katalysator, nicht umgesetztes p-Cresol sowie Spuren von 4-Methylanisol. Diese Lösung wird als Rohstoff zur 4-Methylanisolherstellung nach Ergänzung mit Methanol und p-Cresol vollständig recycelt.

## Patentansprüche

1. Verfahren zur Herstellung von Arylalkylethem, in dem ein Hydroxyaromat mit einem Alkohol in Gegenwart eines Katalysators umgesetzt wird, wobei man ein bei Normaldruck homogenes, Hydroxyaromat, Alkohol und Katalysator enthaltendes Gemisch in den Reaktor einleitet, bei einer Temperatur von 250 - 370°C umsetzt und die Produktkonzentration im Reaktionsgemisch derart einstellt, dass der Katalysator auch während der destillativen Aufarbeitung nicht ausfällt, wobei das Verfahren kontinuierlich durchgeführt wird und das Molverhältnis Katalysator zu Hydroxyaromat 1:32 - 1:60 beträgt.

2. Verfahren nach Anspruch 1, **dadurch gekennzeichnet, dass** man das Molverhältnis Hydroxyaromat zu Alkohol auf 0,5:1, insbesondere etwa 1:1 einstellt.

3. Verfahren nach einem der vorangegangenen Ansprüche, **dadurch gekennzeichnet, dass** der Hydroxyaromat ausgewählt ist aus der Gruppe, bestehend aus Phenol, p-Kresol, 2-Naphtol, Resorcin, Brenzkatechin und Hydrochinon.

4. Verfahren nach einem der vorangegangenen Ansprüche, **dadurch gekennzeichnet, dass** der Alkohol ausgewählt ist aus Methanol, Ethanol, Isopropanol, Butanol, tert.-Butanol, Propanol, Pentanol und/oder Benzylalkohol.

## Claims

1. Process for the preparation of aryl alkyl ethers comprising the step of reacting a hydroxy aromatic with an alcohol in the presence of a catalyst wherein a mixture, homogeneous at atmospheric pressure, comprising a hydroxy aromatic, an alcohol and a catalyst is introduced into a reactor and reacted at a temperature of from 250 to 370°C and the concentration of products in the reaction mixture is adjusted in such a manner that the catalyst does not precipitate even during the distillative working-up, wherein the process is a continuous process and the ratio of catalyst to hydroxyaromatic is 1:32 to 1:60.

2. Process as according to claim 1, **characterized in that** the molar ratio of the hydroxyaromatic to the alcohol is 0.5:1, particularly about 1:1.

3. Process according to claim 1 or 2, **characterized in that** the hydroxyaromatic is selected from the group consisting of phenol, p-cresol, 2-naphthol, resorcinol, pyrocatechol and hydroquinone.

4. Process according to one of the claims 1 to 3, **characterized in that** the alcohol is selected from the group consisting of methanol, ethanol, isopropanol, butanol, tert-butanol, propanol, pentanol and/or benzyl alcohol.

## Revendications

1. Procédé de préparation d'arylalkyléthers, dans lequel un hydroxyaromatique est mis à réagir avec un alcool en présence d'un catalyseur, et dans lequel l'on fait passer un mélange homogène à pression normale, contenant l'hydroxyaromatique, l'alcool et le catalyseur, dans le réacteur, on fait réagir à une température allant de 250 à 370°C et on ajuste la concentration des produits dans le mélange réactionnel de sorte que le catalyseur ne précipite pas, également pendant la séparation par distillation, le procédé étant réalisé en continu et le rapport molaire du catalyseur à l'hydroxyaromatique se situant dans l'intervalle allant de 1:32 à 1:60.

2. Procédé suivant la revendication 1, **caractérisé en ce que** l'on ajuste le rapport molaire de l'hydroxyaromatique à l'alcool à 0,5:1, en particulier 1:1.

3. Procédé selon l'une quelconque des revendications précédentes, **caractérisé en ce que** l'hydroxyaromatique est choisi parmi le groupe consistant en le phénol, le p-crésol, le 2-naphtol, le résorcinol, le benzcatéchol et l'hydroquinone.

4. Procédé selon l'une quelconque des revendications précédentes, **caractérisé en ce que** l'alcool est choisi parmi le méthanol, l'éthanol, l'isopropanol, le butanol, le t-butanol, le propanol, le pentanol et/ou l'alcool benzylique.
